(19) Europäisches Patentamt / European Patent Office / Office européen des brevets

(11) **EP 3 950 701 A1**

(12) **EUROPEAN PATENT APPLICATION**
published in accordance with Art. 153(4) EPC

(43) Date of publication:
**09.02.2022 Bulletin 2022/06**

(21) Application number: **20827192.4**

(22) Date of filing: **15.06.2020**

(51) International Patent Classification (IPC):
*C07K 1/13* (2006.01)       *C07K 16/00* (2006.01)
*C12N 15/115* (2010.01)     *G01N 33/533* (2006.01)

(52) Cooperative Patent Classification (CPC):
**C07K 16/2869; C07K 1/13; C07K 16/32;**
C07K 2317/569

(86) International application number:
**PCT/JP2020/023340**

(87) International publication number:
**WO 2020/255906 (24.12.2020 Gazette 2020/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.06.2019 JP 2019114690**

(71) Applicant: **KONICA MINOLTA, INC.
Tokyo 100-7015 (JP)**

(72) Inventor: **TAKAHASHI, Masaru
Tokyo 100-7015 (JP)**

(74) Representative: **Gille Hrabal
Partnerschaftsgesellschaft mbB
Patentanwälte
Brucknerstraße 20
40593 Düsseldorf (DE)**

(54) **LINKED BODY FOR FLUORESCENT LABELING AGENTS**

(57)    The present invention provides a linked body for fluorescent labeling agents, the linked body suppressing noise due to specific adsorption in performing quantification and localization analysis of a target substance. The present invention is a linked body for fluorescent labeling agents obtained by bonding a probe molecule to a bonding group via a linker, in which a labeling ratio of the bonding group to the probe molecule is 3 or more.

FIG. 1

**Description**

Technical Field

The present invention relates to a linked body for fluorescent labeling agents.

Background Art

Currently, in the field of life science including medicine and pharmacy, development of a labeling agent for labeling a target substance such as an antibody or a cancer marker using a method such as immunohistochemistry, FACS, or ELISA is in progress. Usually, the labeling agent includes a probe molecule such as an antibody exhibiting high affinity with a target substance and a luminescent material that emits light by predetermined excitation light. In particular, study of a fluorescent labeling agent using, as a luminescent material, phosper integrated dots nanoparticles (hereinafter, also referred to as "PID", also translated into English as fluorescent substance-integrated nanoparticles) which are nanoparticles each obtained by integrating a plurality of particles of a phosphor such as an organic fluorescent dye or semiconductor nanoparticles (quantum dots), the phosphor being less likely to be discolored and making observation and imaging for a long time possible, is in progress.

Patent Literature 1 discloses a fluorescent labeling agent containing a probe biological substance bonded to a first bonding group substance via a polymer-derived spacer having a length of 30 angstroms to 1000 angstroms, and phosphor-integrated dots having a second bonding group substance capable of being specifically bonded to the first bonding group substance.

Citation List

Patent Literature

Patent Literature 1: WO 2015/133523 A

Summary of Invention

Technical Problem

By setting the length of the spacer to a predetermined length, the fluorescent labeling agent of Patent Literature 1 suppresses nonspecific adsorption of a substance other than a target substance to probe molecules and phosphor-integrated dots in fluorescent immunostaining, and suppresses generation of noise due to a bright spot other than the target substance to some extent in a fluorescent immunostaining image.

However, in performing quantification and localization analysis of a target substance, for example, when the target substance has a value near a cut-off value where it is difficult to determine whether the target substance is positive or negative, further improvement has been required in order to suppress noise due to non-specific adsorption. In addition, for example, in a fluorescent image displayed for 8 bits using the fluorescent labeling agent of Patent Literature 1, when the luminance per pixel is less than 30, it is not possible to distinguish whether a bright spot is derived from a target substance or derived from non-specific adsorption.

Solution to Problem

As a result of intensive studies on the above problems, the present inventors have found that in a linked body for fluorescent labeling agents in which a probe molecule is bonded to a bonding group via a linker molecule, by setting a labeling ratio of the bonding group to the probe molecule to a predetermined range, non-specific adsorption of a substance other than a target substance to the probe molecule is suppressed, and noise due to a bright spot of a non-specific adsorption substance in a fluorescent immunostaining image can be reduced, thereby completing the present invention. That is, the present invention relates to, for example, the following [1] to [7].

[1] A linked body for fluorescent labeling agents obtained by bonding a probe molecule to a bonding group directly or via a linker molecule, in which a labeling ratio of the bonding group to the probe molecule is 3 or more.

[2] The linked body for fluorescent labeling agents according to [1], in which the labeling ratio of the bonding group to the probe molecule is 20 or less.

[3] The linked body for fluorescent labeling agents according to [1] or [2], in which the linker molecule has a hydrophilic polymer including a structural unit derived from a polyalkylene glycol.

[4] The linked body for fluorescent labeling agents according to [3], in which the polyalkylene glycol is polyethylene glycol or polypropylene glycol.

[5] The linked body for fluorescent labeling agents according to any one of [1] to [4], in which the probe molecule is bonded to the bonding group directly or via a linker molecule having a chain length of more than 0 angstroms and less than 30 angstroms.

[6] The linked body for fluorescent labeling agents according to any one of [1] to [5], in which the bonding group is at least one selected from the group consisting of biotin, avidin, streptavidin, neutravidin, a hapten, and an anti-hapten antibody.

[7] The linked body for fluorescent labeling agents according to any one of [1] to [6], in which the probe molecule is an aptamer or an antibody.

Advantageous Effects of Invention

The present invention can reduce non-specific adsorption of a substance other than a target substance to a probe molecule, and can suppress generation of noise of a bright spot due to a non-specific adsorption substance other than the target substance in a fluorescent immunostaining image.

Brief Description of Drawings

Fig. 1 is a schematic diagram of a linked body for fluorescent labeling agents of the present invention.

Fig. 2 is a schematic diagram of a fluorescent labeling agent in which the linked body for fluorescent labeling agents of the present invention is bonded to a fluorescent material via an affinity substance.

Fig. 3 is a schematic diagram illustrating a state where a fluorescent labeling agent containing the linked body for fluorescent labeling agents of the present invention is bonded to a target substance.

Description of Embodiments

<Linked body for fluorescent labeling agents>

A linked body for fluorescent labeling agents according to the present invention is a linked body for fluorescent labeling agents obtained by bonding a probe molecule to a bonding group directly or via a linker molecule, in which a labeling ratio of the bonding group to the probe molecule is 3 or more. The labeling ratio is preferably 20 or less.

The linked body for fluorescent labeling agents may be any linked body that can make a probe molecule and a target substance bonded to each other directly or indirectly via one or more other bonding molecules (for example, an aptamer or an antibody) between the probe molecule and the target substance without being particularly limited. However, the linked body for fluorescent labeling agents preferably can make the probe molecule and the target substance bonded to each other directly or indirectly via one other bonding molecule.

The linked body for fluorescent labeling agents may be any linked body that can make a bonding group and a fluorescent material bonded to each other directly or indirectly via one or more affinity substances between the probe molecule and the fluorescent material without being particularly limited. However, the linked body for fluorescent labeling agents preferably can make the bonding group and the fluorescent material bonded to each other directly or indirectly via one affinity substance.

As described above, the linked body for fluorescent labeling agents is specifically bonded to a target substance and a fluorescent material to link the target substance and the fluorescent material to each other.

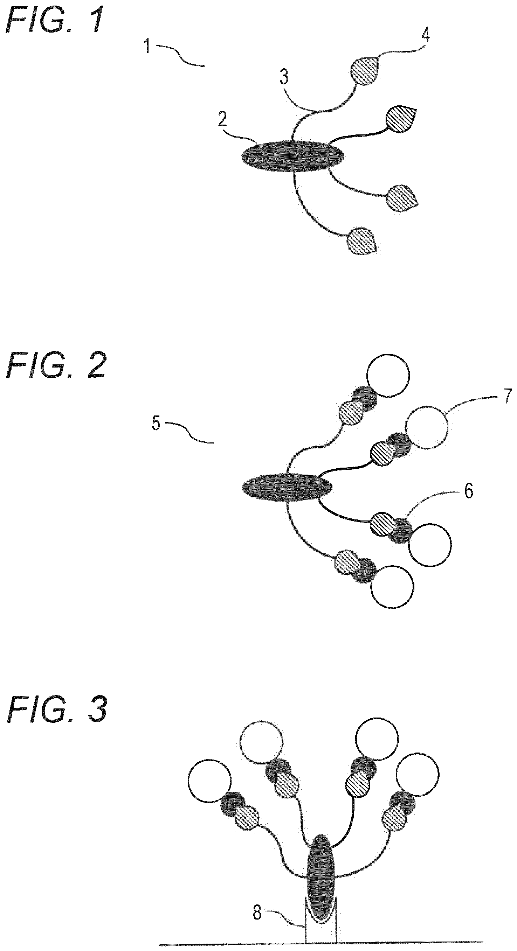

Fig. 1 is an example of a schematic diagram of the linked body for fluorescent labeling agents of the present invention. In the linked body for fluorescent labeling agents illustrated in Fig. 1, a bonding group 4 is bonded to a probe molecule 2 via a linker molecule 3, and a labeling ratio is 4.

<Probe molecule>

The probe molecule may be any molecule that is specifically bonded to a target substance by an antigen-antibody reaction in order to perform measurement by immunoassay, and is preferably selected from the group consisting of an aptamer and an antibody. The aptamer is not particularly limited as long as the aptamer can specifically recognize a target substance by a method similar to an immunostaining method used for an antibody. The aptamer may be a primary aptamer that is specifically bonded to a target substance, a secondary aptamer that is specifically bonded to a primary antibody that is bonded to a target substance or the primary aptamer, or a higher-order aptamer, but is preferably the primary aptamer or the secondary aptamer.

The aptamers can be roughly classified into a nucleic acid (DNA or RNA) aptamer and a peptide aptamer, but both of these can be used as long as these can specifically recognize a target substance or an antibody or a lower-order aptamer bonded to the target substance and can be bonded thereto. The aptamer is preferably a nucleic acid aptamer from a viewpoint of easy availability of oligonucleic acid. The nucleic acid aptamer and the peptide aptamer can be prepared by a known method, or commercially available products can be purchased and used.

The antibody includes not only a whole antibody but also any antibody fragment, derivative, and conjugate. Examples of the antibody include, in addition to the whole antibody, Fab, F(ab')$_2$, CDR, a chimeric antibody, a humanized antibody, a multifunctional antibody, a single chain antibody (scFv), a VHH antibody, and an antibody-drug conjugate. The VHH antibody is an antibody containing a variable region of a heavy chain antibody derived from an animal belonging to a camelid family such as llama, alpaca, or camel, or a cartilage fish such as shark. The antibody can be prepared by a known method, or a commercially available product can be purchased and used.

Examples of the target substance to which the probe molecule is bonded include an amino acid, a protein such as a polypeptide, complexes thereof with a sugar, a lipid, or the like, and a hapten such as DNP, DIG, or FITC. Specific examples of the target substance include a protein cancer marker, a genetic cancer marker, a signal transduction substance, a hormone, an antibody present in a living body, and an artificial antibody (antibody drug) and other administered drugs.

Examples of the cancer marker include an immune protein expressed in a cancer cell, a pathway protein expressed in a cancer cell, and a progression protein expressed in a cancer cell. As these cancer-related proteins, various proteins are known. An appropriate protein can be selected according to a purpose of diagnosis or treatment, a mechanism of action of a drug to be used, and the like without being particularly limited. For example, proteins encoded by genes of an immune gene panel, a pathway gene panel, and a progression gene panel included in a cancer-related gene expression panel provided by nCounter correspond to an immune protein, a pathway protein, and a progression protein expressed in a cancer cell, respectively. In addition, mutant proteins corresponding to mutant genes of these genes can also be included in the immune protein, the pathway protein, and the progression protein, respectively.

Examples of the immune protein expressed in a cancer cell include CD40, TL1A, GITR-L, 4-188-L, CX4D-L, CD70, HHLA2, ICOS-L, CD85, CD86, CD80, MHC-II, PDL1, PDL2, VISTA, BTNL2, B7-H3, B7-H4, CD48, HVEM, CD40L, TNFRSF25, GITR, 4-188, OX40, CD27, TMIGD2, ICOS, CD28, TCR, LAG3, CTLA4, PD1, CD244, TIM3, BTLA, CD160, and LIGHT, which are immune checkpoint proteins.

Examples of the pathway protein expressed in a cancer cell include: EGFR (HER1), HER2, HER3, HER4, IGFR, and HGFR, which are cancer cell growth factors or cancer cell growth factor receptors; VEGF-A, VEGF-B, VEGF-C, VEGF-D, VEGF-E, VEGF-R, P1GF-1, and P1GF-2, which are cell surface antigens, vascular growth factors, or vascular growth factor receptors; interferon, interleukin, G-CSF, M-CSF, EPO, SCF, EGF, FGF, IGF, NGF, PDGF, and TGF, which are cytokines or cytokine receptors; and ER and PgR, which are steroid hormone receptors.

Examples of the progression protein expressed in a cancer cell include ACTG2, ALDOA, APC, BRMS1, CADM1, CAMK2A, CAMK2B, CAMK2D, CCL5, CD82, CDKN1A, CDKN2A, CHD4, CNN1, CST7, CTSL, CXCR2, YBB, DCC, DENR, DLC1, EGLN2, EGLN3, EIF4E2, EIF4EBP1, ENO1, ENO2, ENO3, ETV4, FGFR4, GSN, HK2, HK3, HKDC1, HLA-DPB1, HUNKIL11, KDM1A, KISS1, LDHA, LIFR, MED23, MET, MGAT5, MAP2K4, MT3, MTA1, MTBP, MTOR, MYCL, MYH11, NDRG1, NF2, NFKB1, NME1, NME4, NOS2, NR4A3, PDK1, PEBP4, PFKFB1, PFKFB4, PGK1, PLAUR, PTTG1 RB1, RORB, SET, SLC2A1, SNRPF, SSTR2, TCEB1, TCEB2, TCF20, TF, TLR4, TNFSF10, TP53, TSHR, MMP, MMP2, MMP10, and HIF1, which are cancer progression markers.

Furthermore, examples of the genetic cancer marker include the following nucleic acid molecules. That is, examples of the genetic cancer marker include a naturally occurring nucleic acid such as DNA or RNA (mRNA, tRNA, miRNA, siRNA, non-cording-RNA, and the like), and an artificial nucleic acid such as PNA or LNA (or bridged nucleic acid (BNA)). Specifically, a marker that detects the following genes can be arbitrarily designed with a nucleic acid probe sequence. That is, examples of a gene related to cancer growth and a response ratio of a molecular target drug include HER2, TOP2A, HER3, EGFR, P53, and MET. Furthermore, examples of a gene known as a cancer-related gene include the following genes. Examples of a tyrosine kinase-related gene include ALK, FLT3, AXL, FLT4 (VEGFR3, DDR1, FMS (CSF1R), DDR2, EGFR (ERBB1), HER4 (ERBB4), EML4-ALK, IGF1R, EPHA1, INSR, EPHA2, IRR (INSRR), EPHA3, KIT, EPHA4, LTK, EPHA5, MER (MERTK), EPHA6, MET, EPHA7, MUSK, EPHA8, NPM1-ALK, EPHB1, PDGFRα (PDGFRA), EPHB2, PDGFRβ (PDGFRB), PD-L1, BMI1, LGR5, EPHB3, RET, EPHB4, RON (MST1R), FGFR1, ROS (ROS1), FGFR2, TIE2 (TEK), FGFR3, TRKA (NTRK1), FGFR4, TRKB (NTRK2), FLT1 (VEGFR1), and TRKC (NTRK3).

Examples of a breast cancer-related gene include ATM, BRCA1, BRCA2, BRCA3, CCND1, E-Cadherin, ERBB2, ETV6, FGFR1, HRAS, KRAS, NRAS, NTRK3, p53, and PTEN. Examples of a carcinoid tumor-related gene include BCL2, BRD4, CCND1, CDKN1A, CDKN2A, CTNNB1, HES1, MAP2, MEN1, NF1, NOTCH1, NUT, RAF, SDHD, and VEGFA. Examples of a colorectal cancer-related gene include APC, MSH6, AXIN2, MYH, BMPR1A, p53, DCC, PMS2, KRAS2 (or Ki-ras), PTEN, MLH1, SMAD4, MSH2, STK11, and MSH6. Examples of a lung cancer-related gene include ALK, PTEN, CCND1, RASSF1A, CDKN2A, RB1, EGFR, RET, EML4, ROS1, KRAS2, TP53, and MYC. Examples of a liver cancer-related gene include AxinI, MALAT1, b-catenin, p16 INK4A, c-ERBB-2, p53, CTNNB1, RB1, Cyclin D1, SMAD2, EGFR, SMAD4, IGFR2, TCF1, and KRAS. Examples of a kidney cancer-related gene include Alpha, PRCC, ASPSCR1, PSF, CLTC, TFE3, p54nrb/NONO, and TFEB. Examples of a thyroid cancer-related gene include AKAP10, NTRK1, AKAP9, RET, BRAF, TFG, ELE1, TPM3, H4/D10S170, and TPR. Examples of an ovarian cancer-related gene include AKT2, MDM2, BCL2, MYC, BRCA1, NCOA4, CDKN2A, p53, ERBB2, PIK3CA, GATA4, RB, HRAS, RET, KRAS, and RNASET2. Examples of a prostate cancer-related gene include AR, KLK3, BRCA2, MYC, CDKN1B, NKX3.1, EZH2, p53, GSTP1, and PTEN. Examples of a bone tumor-related gene include CDH11, COL12A1, CNBP, OMD, COL1A1, THRAP3, COL4A5, and USP6.

The antibody drug is a drug containing an antibody (immunoglobulin) that recognizes an antigen as a main component. The antibody drug may be one that has already been put on the market and is used clinically, or a candidate drug used in a clinical test or a clinical trial. Examples of the antibody drug that has been put on the market include humanized anti-HER2 antibody trastuzumab (Herceptin (registered trademark)), anti-CD30 monoclonal antibody brentuximab, anti-VEGF monoclonal antibody bevasizumab, humanized anti-CD33 antibody gemtuzumab, humanized anti-PD-1 antibody pembrolizumab and nibolumab, and an anti-CTLA-4 ipilimumab monoclonal antibody.

The artificial nucleic acid also includes a nucleic acid drug as a detection target. Examples of the nucleic acid drug include decoy, antisense, siRNA, ribozyme, miRNA-mimic, and an aptamer. These can be arbitrarily designed as a molecule targeting a gene such as PDGF-B, C5, MCP-1, SDF-1, or Hepcidin.

<Bonding group>

The bonding group is a molecule that is specifically bonded to an affinity substance bonded to a fluorescent material described later. Examples of the bonding group include biotin, avidin, streptavidin, neutravidin, a hapten, and an anti-hapten antibody. One kind or two or more kinds of these bonding groups may be used. Examples of the hapten include fluorescein isothiocyanate (FITC), digoxigenin (DIG), and dinitrophenol (DNP). Among these compounds, biotin, streptavidin, FITC, and an anti-FITC antibody are preferable from a viewpoint of bonding force. The affinity substance has a contrasting relationship with the bonding group. For example, when the affinity substance is biotin, the bonding group is avidin, streptavidin, or neutravidin, and vice versa. Similarly, when the affinity substance is a hapten, the bonding group is an anti-hapten antibody, and vice versa (see Fig. 2).

As the bonding group, a group in which s bonding group is bonded to a linker molecule in advance may be used. Examples of the group in which a bonding group is bonded to a linker molecule in advance include EZ-LinkTM Maleimide-PEG2-Biotin (Product number: 21901, manufactured by Thermo Fisher Scientific), NHS-dPEG4-Biotin (Product number: 10200, manufactured by Quanta BioDesign Inc.), fluorescein-5 maleimide (Product number: F0810, manufactured by Tokyo Chemical Industry Co., Ltd.), and 5-Carboxyfluorescein N-Succinimidyl Ester (Product number: C2479, manufactured by Tokyo Chemical Industry Co., Ltd.).

<Linker molecule>

In the linked body for fluorescent labeling agents of the present invention, the probe molecule may be bonded to the bonding group via a linker molecule. In addition, the probe molecule may be directly bonded to the bonding group.

The linker molecule may be any molecule that can set the labeling ratio of the bonding group to the probe molecule to 3 or more, preferably 20 or less without being particularly limited, and usually, an organic substance such as a multimer (oligomer) including a dimer and a trimer or a monomer can be used.

The linker molecule preferably has a hydrophilic polymer formed of a structural unit derived from a polyalkylene glycol. Examples of the polyalkylene glycol include polyethylene glycol and polypropylene glycol, and polyethylene glycol (PEG) is more preferable from a viewpoint of easily setting the chain length of the linker molecule according to the number of oxyethylene units.

In addition, as the linker molecule, at least one selected from the group consisting of ficoll, a polyvinyl alcohol, a styrene-maleic anhydride alternating copolymer, a divinyl ether-maleic anhydride alternating copolymer, polyvinyl pyrrolidone, polyvinyl methyl ether, polyvinyl methyl oxazoline, polyethyl oxazoline, polyhydroxypropyl oxazoline, polyhydroxypropyl methacrylamide, polymethacrylamide, polydimethylacrylamide, polyhydroxypropyl methacrylate, polyhydroxyethyl acrylate, hydroxymethyl cellulose, hydroxyethyl cellulose, and polyaspartamide may be used.

The chain length of the linker molecule means the length of a chemical structural portion derived from the linker molecule between a probe molecule and a bonding group described later when the probe molecule and the bonding group are linked to each other. The chain length of the linker molecule can be calculated by a method described in WO 2015/133523 A. For example, when PEG is used as the linker molecule, amino groups are introduced into both ends of the PEG by an amidation reaction, then an amino group at one end is bonded to biotin as a bonding group, a maleimide group is introduced via an amino group at the other end, and the maleimide group is bonded to an antibody as a probe molecule to manufacture the linked body for fluorescent labeling agents of the present invention, the chain length of the linker molecule is a length from a nitrogen atom of the amide bond at one end to an oxygen atom of the amide bond at the other end.

The chain length of the linker molecule can be calculated as an integrated value of a chemical bond distances between atoms. The chemical bond distance can be calculated as an integrated value of a theoretical value of a chemical bond distance between atoms based on self-consistent approach. Calculation is performed assuming that a N-C bond distance is 1.46 angstroms, a C-C bond distance is 1.50 angstroms, and a C-O bond distance is 1.38 angstroms.

The chain length of the linker molecule is usually more than 0 angstroms and less than 30 angstroms. By setting the length of the linker within the above range, the labeling ratio of the bonding group to the probe molecule can be set within a suitable range, which is preferable. In addition, in the above range, non-specific adsorption of a substance other than a target substance to the probe molecules is suppressed, and noise due to a bright spot of a non-specific adsorption substance in a fluorescent immunostaining image can be reduced.

In addition, when the linker molecule is derived from PEG, the length of the linker molecule can be expressed by an oxyethylene unit (n) as a constituent unit of PEG. When the unit is 1 to 5 or the probe molecule is directly bonded the bonding group, non-specific adsorption of a substance other than a target substance to the probe molecule is suppressed, and noise due to a bright spot of a non-specific adsorption substance in a fluorescent immunostaining image can be reduced.

The linker molecule such as PEG can be synthesized by a known method. In addition, for example, a linker molecule having a desired length commercially available from Sigma-Aldrich, Thermo Fisher Scientific, or the like can be purchased. A linker molecule can also be obtained by asking a reagent manufacturer or the like to manufacture the linker molecule by setting the number of repetitions of a chemical structure such as an oxyethylene unit such that the chain length is more than 0 angstroms and less than 30 angstroms.

In addition, a linker molecule to which functional groups (for example, an N-hydroxyester group, a maleimide group, and the like) to be bonded to the bonding group and the probe molecule are bonded in advance may be used.

<Labeling ratio>

The labeling ratio of the bonding group to the probe molecule is a ratio of the bonding group bonded to one probe molecule. The labeling ratio can be calculated, for example, by dividing the concentration of the bonding group of the linked body for fluorescent labeling agents of the present invention by the concentration of the probe molecule, and is 3 or more, preferably 20 or less, and more preferably 10 or less. The labeling ratio can be controlled by adjusting the chain length of the linker molecule.

The concentrations of the bonding group and the probe molecule can be measured by a known method. For example, when the bonding group and the probe molecule have a property of absorbing light having a specific wavelength, the concentrations of the bonding group and the probe molecule can be determined by measuring absorbance. Alternatively, when the bonding group has a property of exhibiting luminescence such as fluorescence, the concentration of the bonding group can be determined by measuring luminescence intensity. The concentration of the probe molecule can be calculated, for example, from the molecular weight of the probe molecule with respect to a solvent used at the time of manufacturing the linked body for fluorescent labeling agents of the present invention. When it is difficult to measure absorbance, for example, the labeling ratio can be determined by performing measurement using a matrix-assisted laser desorption ionization time-of-flight mass spectrometer (MALDI-TOFMS). That is, by the measurement, in the linked body for fluorescent labeling agents in which the probe molecule is bonded to the bonding group, a plurality of peaks derived from the linked body for fluorescent labeling agents corresponding to the number of bonding groups bonded to the probe molecule is usually detected on a higher molecular weight side than the molecular weight of only the probe molecule. Therefore, the labeling ratio can be calculated by multiplying an each peak area ratio with respect to the sum of the peak areas by the number of bonding groups in each peak.

A reason why non-specific adsorption of a substance other than a target substance to the probe molecules is suppressed, and noise due to a bright spot of a non-specific adsorption substance in a fluorescent immunostaining image can be reduced by setting the labeling ratio within the above range is not well known, but it is presumed as follows. In a state where the labeling ratio is moderately high, a large number of bonding groups that are specifically bonded to an affinity substance bonded to a fluorescent material are present, and therefore the probe molecule is covered with a large amount of the fluorescent material. Therefore, non-specific adsorption of a substance other than a target substance to the probe molecules is less likely to occur. However, in a state where the labeling ratio is too high, the probe molecule is covered with a very large amount of the fluorescent material, and therefore the probe molecule is less likely to be bonded to an essential target substance. Meanwhile, when the labeling ratio is low, the amount of the fluorescent material covering the probe molecules is small, and therefore non-specific adsorption of a substance other than a target substance to the probe molecules easily occurs.

<Fluorescent material>

The fluorescent material is not particularly limited as long as the fluorescent material can emit predetermined fluorescence after being bonded to the bonding group of the linked body for fluorescent labeling agents of the present invention directly or indirectly via an affinity substance to form a fluorescent labeling agent, but is preferably formed of phosphor-integrated dots from a viewpoint of being less likely to be discolored and making observation and imaging for a long time possible.

In the present specification, the fluorescent material refers to a general substance that is excited by being irradiated with an X-ray, an ultraviolet ray, a visible ray, a near-infrared ray, or the like from the outside and emits light in a process from an excited state to a ground state. Therefore, the fluorescent material in the present invention may be a substance that emits fluorescence in a narrow sense, which is luminescence caused by deactivation from an excited singlet, or may be a substance that emits phosphorescence, which is luminescence caused by deactivation from a triplet, regardless of a transition mode when returning from an excited state to a ground state.

In the present invention, the fluorescent material is not limited by an emission lifetime after excitation light is blocked. Therefore, a substance known as a phosphorescent substance such as zinc sulfide or strontium aluminate may be used. Such fluorescent materials can be roughly classified into an organic fluorescent material and an inorganic fluorescent material.

The phosphor-integrated dot is a nano-sized particle having a diameter of less than 1 μm and having a structure in which a plurality of particles of a phosphor such as a fluorescent dye is integrated inside or on a surface of matrix particles made of an organic substance or an inorganic substance. The phosphor-integrated dot is preferably a particle capable of emitting fluorescence with sufficient luminance with one phosphor-integrated dot. The phosphor-integrated dots have an average particle size of preferably 40 to 300 nm, more preferably 80 to 150 nm. In addition, the phosphor-integrated dots preferably have a particle size variation coefficient of 5 to 15%. The average particle size and the particle size variation coefficient can be calculated by, for example, observing particles with a scanning electron microscope and measuring the particle sizes of 20 particles.

The phosphor integrated in the phosphor-integrated dots is not particularly limited, but for example, a known organic phosphor dye or semiconductor nanoparticles (also referred to as quantum dots or the like) can be used. Hereinafter, when an organic fluorescent dye is used as the phosphor, the phosphor-integrated dots are referred to as organic fluorescent dye-integrated dots, and when an inorganic phosphor is used as the phosphor, the phosphor-integrated dots are referred to as inorganic phosphor-integrated dots.

As the phosphor, a phosphor that emits light of a predetermined wavelength (color) can be selected according to an application. When there are two or more kinds of target substances, it is possible to select a combination of phosphors that emit fluorescence of different wavelengths corresponding to the target substances, and to prepare phosphor-integrated dots in which each of the phosphors is integrated. In this case, it is preferable to select phosphors whose fluorescence wavelength peaks are separated from each other by 100 nm or more.

[Organic fluorescent dye-integrated dots]

The organic fluorescent dye-integrated dots are preferably formed of a nano-sized fluorescent material obtained by integrating a plurality of particles of an organic fluorescent dye inside or on a surface of a substance serving as a matrix of the particles.

Examples of the organic fluorescent dye include a fluorescent dye formed of a low molecular weight organic compound that is not a high molecular weight organic compound including a polymer, such as a rhodamine-based dye, a pyrromethene-based dye, a fluorescein-based dye, an Alexa Fluor (registered trademark, manufactured by Invitrogen)-based dye, a BODIPY (registered trademark, manufactured by Invitrogen)-based dye, a Cascade (registered trademark, manufactured by Invitrogen)-based dye, a coumarin-based dye, an NBD (registered trademark)-based dye, a pyrene-based dye, a cyanine-based dye, a perylene-based dye, or an oxazine-based dye. Among these dyes, a rhodamine-based dye such as TAMRA (registered trademark), sulforhodamine 101, or Texas Red (registered trademark) that is a hydrochloride of sulforhodamine 101, a pyrromethene-based dye such as pyrromethene 556, and a perylene-based dye such as perylene diimide are preferable because of having high light resistance.

Specific examples thereof include 5-carboxy-fluorescein, 6-carboxy-fluorescein, 5,6-dicarboxy-fluorescein, 6-carboxy-2',4,4',5',7,7'-hexachlorofluorescein, 6-carboxy-2',4,7,7'-tetrachlorofluorescein, 6-carboxy-4',5'-dichloro-2',7'-dimethoxyfluorescein, naphthofluorescein, 5-carboxy-rhodamine, 6-carboxy-rhodamine, 5,6-dicarboxy-rhodamine, rhodamine 6G, tetramethylrhodamine, X-rhodamine, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750, BODIPY FL, BODIPY TMR, BODIPY 493/503, BODIPY 530/550, BODIPY 558/568, BODIPY 564/570, BODIPY 576/589, BODIPY 581/591, BODIPY 630/650, BODIPY 650/665 (manufactured by Invitrogen Corporation), methoxycoumarin, eosin, NBD, pyrene, Cy5, Cy5.5, and Cy7. These compounds may be used singly or in combination of two or more kinds thereof.

Examples of the matrix constituting the organic fluorescent dye-integrated dots include a substance that can integrate an organic fluorescent dye by a physical or chemical bonding force, such as resin or silica. Specifically, a resin such as polystyrene, polyamide, polylactic acid, polyacrylonitrile, polyglycidyl methacrylate, polymelamine, polyurea, polybenzoguanamine, polyfuran, polyxylene, a phenol resin, or an ASA resin (acrylonitrile-styrene-methyl acrylate copolymer), a polysaccharide, or a hydrophobic compound such as silica is preferable. A melamine resin and a styrene resin are more preferable because fluorescent dye-integrated dots can be easily prepared and particles having a high luminescence intensity can be obtained.

For example, organic fluorescent dye-integrated dots prepared by using a fluorescent dye such as TAMRA (registered trademark) or pyrromethene as the phosphor and using a resin such as a melamine resin or silica as the matrix are less likely to be discolored and have a high luminance, and therefore are preferable as the phosphor-integrated dots.

[Inorganic phosphor-integrated dots]

The inorganic phosphor-integrated dots are formed of a nano-sized fluorescent material obtained by integrating a plurality of semiconductor nanoparticles inside or on a surface of a substance serving as a matrix of the particles. Examples of the semiconductor nanoparticles include a quantum dot containing a group II-VI compound, a group III-V compound, or a group IV element, and a quantum dot such as CdSe exemplified in WO 2012/133047 A.

Specific examples thereof include CdSe, CdS, CdTe, ZnSe, ZnS, ZnTe, InP, InN, InAs, InGaP, GaP, GaAs, Si, and Ge, but are not limited thereto. In addition, as the inorganic phosphor, these semiconductor nanoparticles may be used singly or as a mixture of two or more kinds thereof.

As the inorganic phosphor-integrated dot, a quantum dot in which a semiconductor nanoparticle is used as a core and a shell is disposed around the core can be used. When a core/shell is expressed as a notation of the core-shell type semiconductor nanoparticle, examples of the semiconductor nanoparticle having a shell include CdSe/Zn exemplified in WO 2012/133047 A.

In addition, as the inorganic phosphor-integrated dots, semiconductor nanoparticles surface-treated with an organic polymer or the like can be used. Examples of the surface-treated semiconductor nanoparticles include CdSe/ZnS having a particle surface modified with a carboxyl group (manufactured by Invitrogen) and CdSe/ZnS having a particle surface modified with an amino group (manufactured by Invitrogen).

Examples of the matrix constituting the inorganic phosphor-integrated dots include a substance that can integrate an inorganic phosphor with a physical or chemical bonding force, such as a resin or silica. Examples of the resin include: a thermosetting resin such as a melamine resin, a urea resin, a benzoguanamine resin, a phenol resin, or a xylene resin; and various homopolymers and copolymers prepared by using one or more kinds of monomers, such as a styrene resin, a (meth)acrylic resin, polyacrylonitrile, an acrylonitrile-styrene copolymer (AS resin), or an acrylonitrile-styrene-methyl acrylate copolymer (ASA resin).

<Use mode of linked body for fluorescent labeling agents>

The linked body for fluorescent labeling agents of the present invention can be bonded to a target substance via a probe molecule (see Fig. 3). In addition, when a target substance is measured using the linked body for fluorescent labeling agents, the measurement may be performed using a product obtained by bonding the linked body for fluorescent labeling agents to a fluorescent material via an affinity substance to prepare a fluorescent labeling agent and then bonding the fluorescent labeling agent to the target substance via a probe molecule, or may be performed using a product obtained by bonding the linked body for fluorescent labeling agents to the target substance and then bonding the resulting product to a fluorescent material via an affinity substance.

<Manufacture of linked body for fluorescent labeling agents>

In a case where a linked body for fluorescent labeling agents is manufactured, for example, when a probe molecule is directly bonded to a bonding group, when a probe molecule is bonded to a terminal of a linker molecule, and then the other terminal of the linker molecule is bonded to a bonding group, or when a bonding group is bonded to a terminal of a linker molecule, and then the other terminal of the linker molecule is bonded to a probe molecule, a bonding mode thereof is not particularly limited. As the bonding mode, for example, bonding can be performed using an appropriate bonding mode such as a covalent bond, an ionic bond, a hydrogen bond, a coordinate bond, physical adsorption, or chemical adsorption. A covalent bond such as an amide bond, an ester bond, an imide bond, or a bond using thiol addition to a maleimide group is preferable from a viewpoint of the strength of bonding force. Specific examples thereof include a thiol group-maleimide group coupling reaction method, a crosslinking reaction method using a crosslinking agent, and an ionic bonding method.

The bonding group, the linker molecule, and the probe molecule can be bonded to each other by a known method after maleimidation, amination, thiolation, or the like of terminals of these molecules by, for example, the following known method. The maleimidation can be performed by many conventionally known methods such as a method for obtaining a maleimide by a dehydration reaction between maleic anhydride and a primary amine. The amination can be performed, for example, by a reductive amination reaction between an aldehyde or a ketone and a primary amine. The thiolation can be performed, for example, by reducing an intramolecular disulfide bond using an appropriate reducing agent such as dithiothreitol (DTT).

When the bonding group is bonded to the linker molecule via the linker molecule, the order of bonding the bonding group and the probe molecule to the linker is not particularly limited. For example, the probe molecule may be bonded to the linker molecule after the bonding group is bonded to the linker molecule, or the bonding group may be bonded to the linker molecule after the probe molecule is bonded to the linker molecule. In addition, a commercially available linker molecule to which the bonding group is bonded in advance may be obtained, and the probe molecule may be bonded thereto, or a commercially available linker molecule to which the probe molecule is bonded in advance may be obtained, and the bonding group may be bonded thereto.

Examples

Hereinafter, the present invention will be described more specifically based on Examples, but is not limited to these Examples.

[Example 1]

(1) Preparation of reduced antibody solution

An anti-rabbit IgG antibody was used as a probe molecule. 50 μg of an anti-rabbit IgG antibody (LO-RG-1) was dissolved in a 50 mM Tris solution. Thereafter, a dithiothreitol (DTT) solution was added thereto such that a final concentration was 3 mM, and the mixture was mixed. The mixed solution was caused to react at 37°C for 30 minutes, and then treated with a desalting column to purify the reduced anti-rabbit IgG antibody. 200 μL of the purified antibody was dissolved in a 50 mM Tris solution to prepare a reduced antibody solution.

(2) Preparation of bonding group solution

Fluorescein-5 maleimide (Product No.: F0810, manufactured by TCI) was used as a bonding group. To the bonding group, DMSO was added to prepare a 0.4 mM bonding group solution.

(3) Preparation of fluorescein-labeled anti-rabbit IgG antibody solution

The reduced antibody solution prepared in (1) and 8.5 μL of the 0.4 mM bonding group solution prepared in (2) were mixed and caused to react with each other at 37°C for 30 minutes. The reaction solution was put into a desalting column ("Zeba Desalt Spin Colums", manufactured by Thermo Fisher Scientific) to purify a fluorescein-labeled anti-rabbit IgG antibody which is one form of the linked body for fluorescent labeling agents of the present invention. The absorbance of the purified solution at a wavelength of 300 nm was measured using an absorptiometer ("F-7000", manufactured by Hitachi, Ltd.), and the amount of protein in the solution was calculated. From the calculated value, a 250 μg/mL fluorescein-labeled anti-rabbit IgG antibody solution of Example 1 was prepared using a 50 mM Tris solution.

[Example 2]

A 5-carboxyfluorescein-labeled anti-rabbit IgG antibody solution of Example 2 was prepared by a similar method to that in Example 1 except that 5-Caboxyfluorescein N-Succinimidyl Ester (Product number: C2479, manufactured by TCI) was used as the bonding group.

[Example 3]

A biotin-labeled anti-VHH antibody solution of Example 3 was prepared by a similar method to that in Example 1 except that the anti-rabbit IgG antibody was changed to an anti-rabbit VHH antibody (hereinafter, simply referred to as "anti-VHH antibody").

[Example 4]

A biotin-labeled anti-rabbit IgG antibody solution of Example 4 was prepared by a similar method to that in Example 1 except that the bonding group was changed to EZ-LinkTM Maleimide-PEG2-Biotin (Product number: 21901, manufactured by Thermo Fisher Scientific) in which a bonding group was bonded to a linker molecule in advance. The linker had a chain length of 16.1 angstroms.

[Example 5]

A biotin-labeled anti-VHH antibody solution of Example 5 was prepared by a similar method to that in Example 1 except that the anti-rabbit IgG antibody was changed to an anti-VHH antibody and the bonding group was changed to NHS-dPEG4-Biotin (Product number: 10200, manufactured by Quanta BioDesign Inc.) in which a bonding group was bonded to a linker molecule in advance. The linker had a chain length of 25.7 angstroms.

[Comparative Example 1]

A biotin-labeled anti-rabbit IgG antibody solution of Comparative Example 1 was prepared by a similar method to that in Example 1 except that the bonding group was changed to Biotin-PEG6-NH-Mal (Product number: 2461006-250, manufactured by PurePEG) in which a bonding group was bonded to a linker molecule in advance. The linker had a chain length of 33.6 angstroms.

[Comparative Example 2]

A FITC-labeled anti-rabbit IgG antibody solution of Comparative Example 2 was prepared by a similar method to that in Example 1 except that the bonding group was changed to FITC-PEG-Mal, 500 (Product No.: FL04022-500, manufactured by Biochemipeg) in which a bonding group was bonded to a linker molecule in advance. The linker had a chain length of 55.5 angstroms.

[Comparative Example 3]

A biotin-labeled anti-rabbit IgG antibody solution of Comparative Example 3 was prepared by a similar method to that in Example 1 except that the bonding group was changed to EZ-LinkTM Maleimide-PEG 11-Biotin (Product number: 21911, manufactured by Thermo Fisher Scientific) in which a bonding group was bonded to a linker molecule in advance. The linker had a chain length of 55.5 angstroms.

[Comparative Example 4]

A FITC-labeled anti-rabbit IgG antibody solution of Comparative Example 4 was prepared by a similar method to that in Example 1 except that the bonding group was changed to FITC-PEG-Mal, 600 (Product No.: FL04022-600, manufactured by Biochemipeg) in which a bonding group was bonded to a linker molecule in advance. The linker had a chain length of 64.7 angstroms.

[Comparative Example 5]

A biotin-labeled anti-rabbit IgG antibody solution of Comparative Example 5 was prepared by a similar method to that in Example 1 except that the bonding group was changed to Biotin-PEG-Mal, MW 1000 (Product number: PG2-BNML-lk, manufactured by Nanocs Corporation) in which a bonding group was bonded to a linker molecule in advance. The linker had a chain length of 104.7 angstroms.

[Comparative Example 6]

A FITC-labeled anti-rabbit IgG antibody solution of Comparative Example 6 was prepared by a similar method to that in Example 1 except that the bonding group was changed to FITC PEG Maleimide (Product number: PG2-FCML-2k, manufactured by Nanocs Corporation) in which a bonding group was bonded to a linker molecule in advance. The linker had a chain length of 202.0 angstroms.

[Comparative Example 7]

A FITC-labeled anti-rabbit IgG antibody solution of Comparative Example 7 was prepared by a similar method to that in Example 1 except that the bonding group was changed to FITC PEG NHS (Product No.: PG2-FCNS-2k, manufactured by Nanocs Corporation) in which a bonding group was bonded to a linker molecule in advance. The linker had a chain length of 202.0 angstroms.

[Comparative Example 8]

A biotin-labeled anti-VHH antibody solution of Comparative Example 8 was prepared by a similar method to that in Example 1 except that the anti-rabbit IgG body was changed to an anti-VHH antibody and the bonding group was changed to Biotin-PEG-SCM, 2 kDa (Product number: PJK-1900, manufactured by Craative PEG Works) in which a bonding group was bonded to a linker molecule in advance. The linker had a chain length of 202.0 angstroms.

[Comparative Example 9]

A biotin-labeled anti-rabbit IgG antibody solution of Comparative Example 9 was prepared by a similar method to that in Example 1 except that the bonding group was changed to Biotin-PEG-SCM, MW 2000 (Product number: PG2-BNML-2k, manufactured by Nanocs Corporation) in which a bonding group was bonded to a linker molecule in advance. The linker had a chain length of 202.0 angstroms.

[Comparative Example 10]

A biotin-labeled anti-rabbit IgG antibody solution of Comparative Example 10 was prepared by a similar method to that in Example 1 except that the anti-rabbit IgG body was changed to an anti-VHH antibody and the bonding group was changed to Biotin-PEG-NHS (Product number: PG2-BNNS-5K-1, manufactured by Nanocs Corporation) in which a bonding group was bonded to a linker molecule in advance. The linker had a chain length of 494.0 angstroms.

[Comparative Example 11]

A biotin-labeled anti-rabbit IgG antibody solution of Comparative Example 11 was prepared by a similar method to that in Example 1 except that the bonding group was changed to Biotin-PEG-SCM, MW 5000 (Product number: PG2-BNML-5k, manufactured by Nanocs Corporation) in which a bonding group was bonded to a linker molecule in advance. The linker had a chain length of 494.0 angstroms.

[Comparative Example 12]

A biotin-labeled anti-rabbit IgG antibody solution of Comparative Example 12 was prepared by a similar method to that in Example 1 except that the bonding group was changed to Biotin-PEG-SCM, MW 10000 (Product number: PG2-BNML-10k, manufactured by Nanocs Corporation) in which a bonding group was bonded to a linker molecule in advance. The linker had a chain length of 980.7 angstroms.

<Evaluation of labeling ratio>

(1) Evaluation of labeling ratio of biotin-labeled antibody

The biotin-labeled anti-rabbit IgG antibody/anti-VHH antibody solutions of Example 3 to 5 and Comparative Examples 1, 3, 5, and 8 to 12 were subjected to mass spectrometry using MALDI-TOFMS ("BRUKER autoflex speed", manufactured by Hitachi High-Tech Science Corporation). A peak detected on a higher molecular weight side than the molecular weight of only the antibody as a probe molecule is a peak derived from the number of bonded biotin molecules of the biotin-labeled antibody. Therefore, each peak area ratio with respect to the sum of the peak areas derived from biotin-labeled antibodies was multiplied by the number of biotin molecules of each peak, and an average value for the peaks was taken as a labeling ratio. Table 1 illustrates results thereof.

(2) Evaluation of labeling ratio of fluorescein/FITC-labeled antibody

The absorbance of each of the fluorescein/FITC-labeled anti-rabbit IgG antibody/anti-VHH antibody solutions of Examples 1 and 2 and Comparative Examples 2, 4, 6, and 7 was measured at an absorption wavelength of FITC of 493 nm and an absorption wavelength of protein (anti-rabbit IgG antibody/anti-VHH antibody) of 280 nm using an ultraviolet-visible spectrophotometer ("NanoDrop One", manufactured by Thermo Fisher Scientific). The absorbance at each wavelength was applied to the following formulae (1) and (2), and the concentrations of the fluorescein/FITC and the anti-rabbit IgG antibody/anti-VHH antibody were calculated. The concentration of the fluorescein/FITC was divided by the anti-rabbit IgG antibody/anti-VHH antibody to calculate a concentration ratio, which was taken as a labeling ratio. Table 1 illustrates results thereof.

$$Cab = (A280 - (A493 \times CF))/\varepsilon ab \ldots (1)$$

$$Cdye = A493/\varepsilon dye \ldots (2)$$

(in which C represents a concentration, A represents an absorbance measurement value, $\varepsilon$ represents a molar absorbance coefficient, CF represents a correction coefficient, ab represents an anti-rabbit IgG antibody or an anti-VHH antibody, and dye represents fluorescein or FITC.)

[Table 1]

| | Target substance | Reaction system | Linker molecule | | Probe molecule | | Bright spot caused by non-specific adsorption |
|---|---|---|---|---|---|---|---|
| | | | Number of PEG units | Chain length (Å) | Antibody | Labeling ratio | |
| Example 1 | PgR | Maleimide-FITC | 0 | 0 | LO-RG-1 (Anti rabbit IgG) | 3.42 | 0.23 |
| Example 2 | PgR | NHS-FITC | 0 | 0 | LO-RG-1 (Anti rabbit IgG) | 3.73 | 0.15 |
| Example 3 | HER2 | Maleimide-Biotin | 0 | 0 | VHH (Anti rabbit IgG) | 9.06 | 0.07 |
| Example 4 | HER2 | Maleimide-Biotin | 2 | 16.1 | LO-RG-1 (Anti rabbit IgG) | 3.07 | 0.20 |
| Example 5 | HER2 | NHS-Biotin | 4 | 25.7 | VHH (Anti rabbit IgG) | 7.10 | 0.10 |
| Comparative Example 1 | HER2 | NHS-Biotin | 6 | 33.6 | LO-RG-1 (Anti rabbit IgG) | 2.99 | 0.30 |
| Comparative Example 2 | PgR | Maleimide-FITC | 11 | 55.5 | LO-RG-1 (Anti rabbit IgG) | 1.64 | 0.68 |
| Comparative Example 3 | HER2 | Maleimide-Biotin | 11 | 55.5 | LO-RG-1 (Anti rabbit IgG) | 2.88 | 0.30 |
| Comparative Example 3 | PgR | Maleimide-FITC | 13 | 64.7 | LO-RG-1 (Anti rabbit IgG) | 2.85 | 0.37 |
| Comparative Example 5 | HER2 | Maleimide-Biotin | 22 | 104.7 | LO-RG-1 (Anti rabbit IgG) | 2.65 | 0.40 |

(continued)

| | Target substance | Reaction system | Linker molecule | | Probe molecule | | Bright spot caused by non-specific adsorption |
|---|---|---|---|---|---|---|---|
| | | | Number of PEG units | Chain length (Å) | Antibody | Labeling ratio | |
| Comparative Example 6 | PgR | Maleimide-FITC | 44 | 202 | LO-RG-1 (Anti rabbit IgG) | 2.68 | 0.42 |
| Comparative Example 7 | PgR | NHS-FITC | 44 | 202 | LO-RG-1 (Anti rabbit IgG) | 2.03 | 0.58 |
| Comparative Example 8 | HER2 | NHS-Biotin | 44 | 202 | VHH (Anti rabbit IgG) | 5.20 | 0.58 |
| Comparative Example 9 | HER2 | Maleimide-Biotin | 44 | 202 | LO-RG-1 (Anti rabbit IgG) | 2.19 | 0.80 |
| Comparative Example 10 | HER2 | NHS-Biotin | 111 | 494 | VHH (Anti rabbit IgG) | 1.30 | 1.56 |
| Comparative Example 11 | HER2 | Maleimide-Biotin | 111 | 494 | LO-RG-1 (Anti rabbit IgG) | 0.80 | 0.80 |
| Comparative Example 12 | HER2 | Maleimide-Biotin | 222 | 980.7 | LO-RG-1 (Anti rabbit IgG) | 0.19 | 0.60 |

From the results of Examples 1 to 5 in Table 1, it is considered that regardless of the kinds of probe molecule and bonding group constituting the linked body for fluorescent labeling agents of the present invention, the linked body having a labeling ratio of 3 or more has a small number of bright spots caused by non-specific adsorption of less than 0.3, and is less likely to cause non-specific adsorption by a substance other than a target substance. Note that from Examples 1 to 5, it is found that in the linked body having a labeling ratio of 3 or more, the chain length of the linker molecule is 0 angstroms (state in which a probe molecule is directly bonded to a bonding group), or more than 0 angstroms and less than 30 angstroms.

In contrast, from the results of Comparative Examples 1 to 12 in Table 1, it is considered that regardless of the kinds of probe molecule and bonding group constituting the linked body for fluorescent labeling agents, the linked body having a labeling ratio of less than 3 has a large number of bright spots caused by non-specific adsorption of 0.3 or more, and is likely to cause non-specific adsorption by a substance other than a target substance. Note that from Comparative Examples 1 to 12, it is found that in the linked body having a labeling ratio of less than 3, the chain length of the linker molecule is 30 angstroms or more.

<Fluorescent immunostaining 1>

Using the biotin-labeled anti-rabbit IgG antibodies and the like prepared in Examples 3 to 5 and Comparative Examples 1, 3, 5, and 8 to 12, tissue immunostaining and morphological observation staining of human breast tissues were performed as follows.

(1) Deparaffinization step

As a tissue section for staining, a tissue array slide of HER2 (-) ("CB-A712 series", manufactured by Cosmo Bio Co., Ltd.) was used, and the slide was deparaffinized. The HER2 score "-" indicates a tissue section defined as a score "0" in the HER2 guidelines (third edition) prepared by the Trastuzumab pathology committee. Specifically, the HER2 score "-" indicates a tissue section confirmed to be "no positive reaction to cell membrane" by a DAB method among tissue sections corresponding to "cancer cells with no positive staining in cell membrane or positive staining in cell membrane < 10% (positive staining localized to cell membrane is not to be determined)".

(2) Activation step

The tissue array slide obtained in (1) was subjected to displacement washing with water, and then autoclaved at 121°C for 15 minutes in a 10 mM citrate buffer solution (pH 6.0) to activate an antigen. Thereafter, the tissue array slide was washed with PBS, and the washed tissue array slide was blocked using PBS containing 1% BSA for one hour.

(3) Immunostaining

(3-1) Primary reaction

The tissue array slide obtained in (2) was treated with a solution prepared by adjusting an anti-HER2 rabbit monoclonal antibody ("4B5", manufactured by Ventana Corporation) to 0.05 nM using PBS containing 1% BSA, and a reaction was caused overnight at 4°C.

(3-2) Secondary reaction

The tissue array slide that had been subjected to the primary reaction was washed with PBS, and then caused to react with the biotin-labeled anti-rabbit IgG antibody and the like diluted to 6 μg/mL with PBS containing 1% BSA or the like at room temperature for 30 minutes.

(4) Morphological observation staining (hematoxylin-eosin staining)

The slide after the secondary reaction was washed with PBS, and then the tissue array slide was subjected to hematoxylin staining using a Mayer's hematoxylin solution for five minutes. The tissue array slide was washed with running water at 45°C for three minutes, and then subjected to eosin staining using a 1% eosin solution for five minutes.

(5) Mounting

After staining in (4), an operation of immersing the tissue array slide in pure ethanol for five minutes was performed four times to perform washing and dehydration. Next, an operation of immersing the slide in xylene for five minutes was performed four times to perform clearing. Finally, a tissue ride slide was mounted using a mounting medium ("Entellan new", manufactured by Merck Millipore) to obtain an observation slide using a HER2 antigen as a target substance.

<Fluorescent immunostaining 2>

An observation slide using a PgR antigen as a target substance was obtained by a similar method to that in <Fluorescent immunostaining 1> except that the biotin-labeled anti-rabbit IgG antibody and the like used in the <Fluorescent immunostaining 1> were changed to fluorescein-labeled anti-rabbit IgG antibodies and the like prepared in Examples 1 and 2 and Comparative Examples 2, 4, 6, and 7, and the tissue array slide of HER2 (-) was changed to tissue array slides of progesterone receptor (PgR) (-) and (+) ("Posicon slide series", manufactured by Pathology Institute Corp.).

<Measurement of bright spot caused by non-specific adsorption>

A state where the observation slide was irradiated with predetermined excitation light to emit fluorescence was observed and imaged with a fluorescence microscope ("BX-53", manufactured by Olympus Corporation) and a digital camera for a microscope ("DP73", manufactured by Olympus Corporation). The excitation light was caused to pass through an optical filter and was thereby set to 575 to 600 nm. In addition, fluorescence to be observed was also caused to pass through an optical filter and was thereby set to a wavelength (nm) of 612 to 692 nm. As conditions of the excitation wavelength at the time of microscopic observation and image acquisition, irradiation energy around the center of a visual field was set to 900 W/cm$^2$ in excitation of 580 nm.

Exposure time at the time of image acquisition was set arbitrarily (for example, set to 20 ms) so as not to saturate the luminance of an image, and imaging was performed. The number of bright spots of the tissue of HER2 (-) was taken as an average value of 1000 cells measured by an ImageJ FindMaxims method based on an image imaged at magnification of 400. The number of bright spots of the tissue of PgR(-) was taken as an average value of 1000 cells measured by an ImageJ FindMaxims method based on an image imaged at magnification of 40. Table 1 illustrates results thereof.

<Measurement of bright spot caused by target substance>

Observation and imaging were performed on the observation slides of Examples 1, 2, and 4 and Comparative Examples 2, 4, 6 to 8, and 10 by a similar method to that in <Measurement of bright spot caused by non-specific adsorption>.

Exposure time at the time of image acquisition was set arbitrarily (for example, set to 20 ms) so as not to saturate the luminance of an image, and imaging was performed. The number of bright spots of the tissue of PgR (+) was taken as an average value of 1000 cells measured by an ImageJ FindMaxims method based on an image imaged at magnification of 40. Furthermore, a value obtained by dividing the number of bright spots of the tissue of PgR (+) by the number of bright spots of the tissue of PgR (-) was calculated as detection sensitivity. Table 2 illustrates results thereof.

[Table 2]

| | Target substance | Reaction system | Linker molecule | | Probe molecule | | Caused by non-specific adsorption | | Caused by target substance' | | Detection sensitivity | |
|---|---|---|---|---|---|---|---|---|---|---|---|---|
| | | | Number of PEG units | Chain length (Å) | Antibody | Labeling ratio | Number of bright spots | Evaluation | Number of bright spots | Evaluation | Number of bright spots | Evaluation |
| Example 1 | PgR | Maleimide-FITC | 0 | 0 | LO-RG-1 (Anti rabbit IgG) | 3.42 | 0.23 | ◎ | 25.19 | ◎ | 109 | ◎ |
| Example 2 | PgR | NHS-FITC | 0 | 0 | LO-RG-1 (Anti rabbit IgG) | 3.73 | 0.15 | ◎ | 22.68 | ◎ | 149 | ◎ |
| Example 4 | HER2 | NHS-Biotin | 4 | 25.7 | VHH (Anti rabbit IgG) | 7.10 | 0.10 | ◎ | 437.50 | ◎ | 4297 | ◎ |
| Comparative Example 2 | PgR | Maleimide-FITC | 11 | 55.5 | LO-RG-1 (Anti rabbit IgG) | 1.64 | 0.68 | × | 22.21 | ◎ | 33 | o |
| Comparative Example 4 | PgR | Maleimide-FITC | 13 | 64.7 | LO-RG-1 (Anti rabbit IgG) | 2.85 | 0.37 | o | 18.09 | o | 48 | o |
| Comparative Example 6 | PgR | Maleimide-FITC | 44 | 202 | LO-RG-1 (Anti rabbit IgG) | 2.68 | 0.42 | o | 14.34 | o | 34 | o |
| Comparative Example 7 | PgR | NHS-FITC | 44 | 202 | LO-RG-1 (Anti rabbit IgG) | 2.03 | 0.58 | × | 8.34 | × | 14 | × |

(continued)

| | Target substance | Reaction system | Linker molecule | | Probe molecule | | Caused by non-specific adsorption | | Caused by target substance' | | Detection sensitivity | |
|---|---|---|---|---|---|---|---|---|---|---|---|---|
| | | | Number of PEG units | Chain length (Å) | Antibody | Labeling ratio | Number of bright spots | Evaluation | Number of bright spots | Evaluation | Number of bright spots | Evaluation |
| Comparative Example 8 | HER2 | NHS-Biotin | 44 | 202 | VHH (Anti rabbit IgG) | 5.20 | 0.58 | × | 213.50 | × | 160 | × |
| Comparative Example 10 | HER2 | NHS-Biotin | 111 | 494 | VHH (Anti rabbit IgG) | 1.30 | 1.56 | × | 139.30 | × | 89 | × |

Evaluation criterion for HER2 and PgR bright spots caused by non-specific adsorption (n: number of bright spots): ◎ n ≤ 0.3, o 0.3 < n ≤ 0.5, × 0.5 < n
Evaluation criterion for HER2 bright spots caused by target substance (n: number of bright spots): ◎ n > 400, ∘ 400 ≥ n > 300, × 300 ≥ n
Evaluation criterion for PgR bright spots caused by target substance (n: number of bright spots): ◎ n > 20, o 20 ≥ n > 10, × 10 ≥ n
Evaluation criterion for detection sensitivity of HER2 (n: number of bright spots): ◎ n > 1333, ∘ 1333 ≥ n > 600, × 600 ≥ n
Evaluation criterion for detection sensitivity of PgR (n: number of bright spots): ◎ n > 66, o 66 ≥ n > 20, × 20 ≥ n

Table 2 indicates that in a case where the same anti-rabbit IgG antibody was used for detection of PgR as a target substance, when the labeling ratio was 3 or more as in Examples 1 and 2, not only the number of bright spots caused by non-specific adsorption was decreased, but also the number of bright spots caused by the target substance was increased, and the detection sensitivity was improved as compared with the case where the labeling ratio was less than 3 as in Comparative Examples 2, 4, 6, and 7.

A similar result was indicated in comparison between Example 4 and Comparative Examples 8 and 10 when the same anti-VHH antibody was used for detection of HER2 as a target substance.

Reference Signs List

1    Linked body for fluorescent labeling agents
2    Probe molecule
3    Linker molecule
4    Bonding group
5    Fluorescent labeling agent
6    Affinity substance
7    Fluorescent material
8    Target substance

**Claims**

1. A linked body for fluorescent labeling agents obtained by bonding a probe molecule to a bonding group directly or via a linker molecule, wherein
a labeling ratio of the bonding group to the probe molecule is 3 or more.

2. The linked body for fluorescent labeling agents according to claim 1, wherein the labeling ratio of the bonding group to the probe molecule is 20 or less.

3. The linked body for fluorescent labeling agents according to claim 1 or 2, wherein the linker molecule has a hydrophilic polymer including a structural unit derived from a polyalkylene glycol.

4. The linked body for fluorescent labeling agents according to claim 3, wherein the polyalkylene glycol is polyethylene glycol or polypropylene glycol.

5. The linked body for fluorescent labeling agents according to any one of claims 1 to 4, wherein the probe molecule is bonded to the bonding group directly or via a linker molecule having a chain length of more than 0 angstroms and less than 30 angstroms.

6. The linked body for fluorescent labeling agents according to any one of claims 1 to 5, wherein the bonding group is at least one selected from the group consisting of biotin, avidin, streptavidin, neutravidin, a hapten, and an anti-hapten antibody.

7. The linked body for fluorescent labeling agents according to any one of claims 1 to 6, wherein the probe molecule is an aptamer or an antibody.

*FIG. 1*

*FIG. 2*

*FIG. 3*

## INTERNATIONAL SEARCH REPORT

| International application No. |
| --- |
| PCT/JP2020/023340 |

A. CLASSIFICATION OF SUBJECT MATTER
C07K 1/13(2006.01)i; C07K 16/00(2006.01)i; C12N 15/115(2010.01)i; G01N 33/533(2006.01)i
FI: G01N33/533; C12N15/115 Z; C07K16/00; C07K1/13
According to International Patent Classification (IPC) or to both national classification and IPC

B. FIELDS SEARCHED

Minimum documentation searched (classification system followed by classification symbols)
C07K1/13; C07K16/00; C12N15/115; G01N33/533

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); Scopus

C. DOCUMENTS CONSIDERED TO BE RELEVANT

| Category* | Citation of document, with indication, where appropriate, of the relevant passages | Relevant to claim No. |
| --- | --- | --- |
| X | AAT BIOQUEST, ReadiLink TM Protein Biotinylation Kit, catalogue, no. 5520, 2014, pp. 1-2, [online], 2014 [retrieval date 06 August 2020], internet:<URL:https://docs.aatbio.com/products/pro tocol-and-product-informaton-sheet-pis/protocol-for-readilink-pratein-biotinylation-kit-version-4b3756b08f.pdf> p. 1, paragraph [0001], fig. 1 | 1-2, 5-7 |
| X | WO 2005/075997 A1 (ASAHI KASEI CORPORATION) 18.08.2005 (2005-08-18) paragraphs [0044]-[0045] | 1-4, 6-7 |
| Y | paragraphs [0044]-[0045] | 5 |
| Y | THERMO SCIENTIFIC, Avidin-Biotin Technical Handbook, catalogue, no.1601675, THERMO FISHER SCIENTIFIC INC., 2009, pp. 12-13, [online], 2009 [retrieval date 06 August 2020], internet:<URL:https://www.thermofisher.com/content /dam/LifeTech/Images/integration/1601675_AvBi_HB_I NTL.pdf> pp. 12-13 | 5 |

☒ Further documents are listed in the continuation of Box C.    ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 11 August 2020 (11.08.2020) | 25 August 2020 (25.08.2020) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2020/023340

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2015/146896 A1 (KONICA MINOLTA, INC.) 01.10.2015 (2015-10-01) paragraphs [0054], [0095] | 1-7 |
| A | WO 2015/133523 A1 (KONICA MINOLTA, INC.) 11.09.2015 (2015-09-11) paragraphs [0141], [0143], [0146] | 1-7 |

Form PCT/ISA/210 (continuation of second sheet) (January 2015)

**INTERNATIONAL SEARCH REPORT**

Information on patent family members

International application No.

PCT/JP2020/023340

| Patent Documents referred in the Report | Publication Date | Patent Family | Publication Date |
|---|---|---|---|
| WO 2005/075997 A1 | 18 Aug. 2005 | US 2008/0131978 A1 paragraphs [0100]-[0101] EP 1712915 A1 | |
| WO 2015/146896 A1 | 01 Oct. 2015 | US 2017/0016911 A1 paragraphs [0082], [0141] EP 3124969 A | |
| WO 2015/133523 A1 | 11 Sep. 2015 | US 2017/0074886 A1 paragraphs [0153], [0155], [0158] EP 3115783 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015133523 A

- WO 2012133047 A